# FASCICULE DE BREVET EUROPEEN

(11) **EP 2 266 530 B1**
(45) Date de publication et mention de la délivrance du brevet: **19.10.2016**
(21) Numéro de dépôt: 10171498.8
(22) Date de dépôt: 25.11.2003
(51) Int. Cl.: A61K 8/49, A61K 31/421, A61Q 19/00, A61Q 19/08

(54) **Oxazolidinones pour une utilisation cosmétique cutanée**
Oxazolidinone für eine kosmetische kutane Verwendung
Oxazolidinones for cosmetic cutaneous use

(30) Priorité: 25.11.2002 FR 0214791
(43) Date de publication de la demande: 29.12.2010
(62) Demande divisionnaire de: 03789496.1
(73) Titulaire: Laboratoires Expanscience, 92400 Courbevoie (FR)
(72) Inventeur: Piccirilli, Antoine, 78670, VILLENNES SUR SEINE (FR); Msika, Philippe, 78000, VERSAILLES (FR); Piccardi, Nathalie, 21310, ARCEAU (FR)
(74) Mandataire: Regimbeau

(56) Documents cités:
- EP-A- 0 835 650

## Description

La présente invention se rapporte au traitement cosmétique notamment dermatologique, de la peau et/ou des muqueuses.

L'invention a pour objet une méthode de traitement cosmétique des peaux et/ou des muqueuses sensibles, intolérantes, âgées, présentant des rougeurs cutanées, ou présentant un déséquilibre immunologique non pathologique lié au vieillissement intrinsèque, extrinsèque ou hormonal, consistant à appliquer sur la peau et/ou les muqueuses au moins une oxazolidinone.

Les oxazolidinones selon la présente invention permettent d'inhiber la migration des cellules impliquées dans la réponse immunitaire et/ou inflammatoire et/ou irritative telles que les monocytes, les lymphocytes, les dendrocytes dermiques, les cellules dendritiques et plus particulièrement les cellules de Langerhans suite à un stimulus extérieur ou « signal danger » d'origine chimique, physique, biologique et plus particulièrement immunitaire, ou par suite au vieillissement intrinsèque (chronologique), hormonal ou extrinsèque (soleil, pollution), dont l'intensité serait suffisamment importante pour induire une perturbation de l'homéostasie cutanée.

Une des principales fonctions de la peau est la protection de l'organisme contre des agressions du milieu extérieur. Cette protection est assurée en grande partie grâce à la coopération de cellules présentes dans la peau qui sont capables, en présence d'un agent nocif, de générer une réponse inflammatoire et/ou immunitaire dirigée contre l'agent nocif. Il s'agit des cellules dendritiques, notamment des cellules de Langerhans (CL) de l'épiderme, des dendrocytes dermiques, des monocytes, des lymphocytes, des kératinocytes, des mastocytes, et des cellules endothéliales vasculaires.

Les CL sont des cellules dendritiques issues de la moelle épinière et qui résident dans les tissus non lymphoïdes tels que la peau et les muqueuses (bouche, poumon, vessie, rectum, vagin). Dans la peau, les CL s'intercalent entre les kératinocytes épidermiques en position suprabasale. Sur le plan ultrastructural, elles sont caractérisées par la présence d'un organite spécifique d'origine membranaire, le granule de Birbeck. Sur le plan immunohistochimique, les CL expriment notamment la molécule CD1a et les molécules du Complexe Majeur d'Histocompatibilité de classe II.

Les CL jouent un rôle déterminant dans l'immunité, en tant que cellules présentatrices de l'antigène. En effet, des expériences menées chez la souris démontrent que les CL capturent les antigènes présents au niveau de l'épiderme et migrent vers les tissus lymphoïdes drainant la peau, où elles présentent l'antigène aux cellules T. L'initiation de la réponse immune cutanée dépend de la capacité des CL à quitter l'épiderme pour migrer jusqu'aux ganglions proximaux. Différents facteurs peuvent influencer cette migration : l'expression de molécules d'adhérence, les protéines de la matrice extracellulaire, des haptènes, des cytokines, etc. Néanmoins, les mécanismes impliqués dans la migration des CL ne sont pas totalement encore élucidés. En particulier, avant d'atteindre les ganglions lymphatiques, les CL doivent non seulement traverser la jonction dermo-épidermique (JDE) mais également se frayer un chemin au travers de la matrice extracellulaire (MEC) dermique. La JDE est principalement composée de laminine 5, de collagènes de type IV et VII, de nidogène et de perlecan. La MEC qui entoure les fibroblastes du derme contient essentiellement des collagènes de type I et III.

La maturation, ainsi que l'initiation et la régulation de la migration des CL, sont sous la dépendance de cytokines pro-inflammatoires telles que l'IL-1β (interleukine-1-beta) et le TNF-α (Tumor Necrosis-alpha). Il en résulte que toute agression cutanée, plus particulièrement toute réaction inflammatoire et/ou irritative, capable d'induire en quantité suffisante l'une ou l'autre de ces cytokines ou les deux, est capable de stimuler la migration des CL, et donc de faciliter la réaction allergique si ces CL sont associées à un antigène.

Des pathologies de type dermatologique peuvent être observées comme résultant de la migration des CL suite à la capture d'un antigène de surface. Dans l'eczéma atopique, les CL sont capables de fixer des IgE en surface et d'induire une réponse immunitaire pathologique. Dans l'eczéma de contact, les CL jouent un rôle central puisqu'elles captent et traitent l'antigène avant de le présenter aux lymphocytes T. Celui-ci va le garder en mémoire et la réaction immunitaire sera déclenchée au deuxième contact.

Compte tenu de ce qui précède, il est hautement souhaitable de pouvoir modifier la capacité migratoire des dendrocytes dermiques, des monocytes, des lymphocytes, des cellules de Langerhans (CL), pour tenter d'augmenter le seuil de tolérance ou de limiter la réactivité de la peau allergique et/ou inflammatoire et/ou irritée et/ou âgée. C'est le problème que se propose de résoudre la présente invention. Les inventeurs ont en effet démontré de manière tout à fait surprenante et inattendue que des composés tels que les oxazolidinones permettent d'inhiber de manière spectaculaire la migration des cellules, telles que les cellules de Langerhans, notamment la migration induite par la présence d'un agent allergène.

La demande EP 835 650 décrit un effet physique anti-pénétration des oxazolidinones mais est muet sur leur effet biologique d'inhibition de la migration des cellules de Langerhans.

Selon la présente invention l'oxazolidinone est de formule générale (I) : 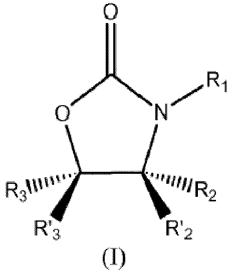 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation de l'invention, R₁ représente un atome d'hydrogène.

Selon un autre mode de réalisation de l'invention, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et/ou R'₂ représente un atome d'hydrogène.

Selon un autre mode de réalisation de l'invention, R₃ et R'₃ représentent un atome d'hydrogène.

Dans un mode de réalisation particulier de la présente invention, R₁ représente un atome d'hydrogène, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et R'₂, R₃ et R'₃ représentent un atome d'hydrogène.

Avantageusement selon la présente invention, l'oxazolidinone est choisie dans le groupe constitué par la 4-dodécyl-oxazolidin-2-one, la 3,4-didodécyl-oxazolidin-2-one et la 4,5-didodécyl-oxazolidin-2-one.

La 4-dodécyl-oxazolidin-2-one peut être représentée par la formule suivante :

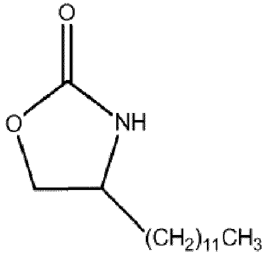

La 3,4-didodécyl-oxazolidin-2-one peut être représentée par la formule suivante :

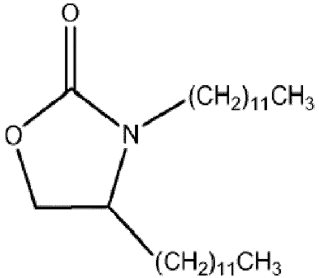

La 4,5-didodécyl-oxazolidin-2-one peut être représentée par la formule suivante :

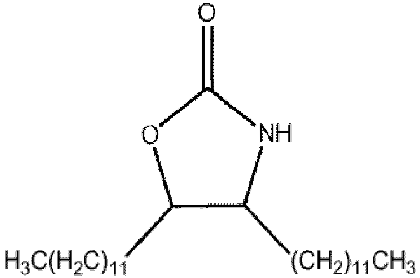

Selon un mode de réalisation de l'invention, la composition comprend en outre au moins un inhibiteur de la migration des cellules, telles que les cellules de Langerhans, sélectionné dans le groupe des inhibiteurs de métalloprotéases matricielles (MMPs).

Par « composés inhibiteurs des métalloprotéases matricielles (MMPs) », on entend selon l'invention tout composé connu de l'homme du métier pour sa capacité d'inhiber l'activité de dégradation de la matrice extracellulaire par les MMPs. Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysines. Elles peuvent être synthétisées par différents types cellulaires au niveau de la peau (fibroblastes, kératinocytes, macrophages, cellules endothéliales, éosinophiles, cellules de Langerhans, etc.). Le groupe des MMPs est ainsi constitué de quatre sous-classes : (1) les collagénases, (2) les gélatinases, (3) les stromélysines et (4) les MMP de type membranaires (MT-MMPs). L'activité des MMPs peut être modulée par des inhibiteurs de protéinase naturellement présents tels que les inhibiteurs tissulaires de métalloprotéinases (TIMPs, notamment les TIMP-1 et TIMP-2). En particulier, le composé actif pour inhiber la migration des cellules de Langerhans est un composé inhibiteur d'au moins une MMP choisie dans le groupe constitué par les MMP-1, MMP-2, MMP-3 MMP-9, MMP-7, MMP-13 et MMP-18. Comme «composé inhibiteur des MMPs », en tant que composé actif pour inhiber la migration des cellules de Langerhans selon la présente invention, on entend en particulier les inhibiteurs tissulaires de métalloprotéinases (TIMPs), l'alpha-2-macroglobuline, les inhibiteurs de l'activateur du plasminogène, la bryostatine-1, les antibiotiques (doxycyclines, minocyclines, etc.), les peptides synthétiques ou naturels ayant une structure similaire aux substrats des MMPs (batimastat, marimastat, etc.), les rétinoïdes (en particulier les rétinoïdes non aromatiques tels que le rétinaldéhyde, la trétinoïne, et l'acide rétinoïque 9-cis, la vitamine A, les rétinoïdes monoaromatiques tels que l'étrétinate, l'alltrans acitrétine et le motrérinide, et les rétinoïdes polyaromatiques tels que l'adapalène, le tazarotène, le tamibarotène et l'arotinoïde methyl sulfone), les anti-oxydants (les piégeurs d'oxygène singulet, etc.), les anti-cancéreux (ou « anti-métastatiques »), les hydrolysats de malt tels que Colalift commercialisés par la société Coletica, les extraits d'algues marines tels que Kelpadélie commercialisés par la société Secma, les extraits de cartilage de requin tels que le complexe MDI commercialisés par la société Atrium, les peptides de riz comme par exemple Colhibin commercialisé par la société Pentapharm, et les extraits peptidiques de lupin. Plus particulièrement, le composé inhibiteur des MMPs selon la présente invention est choisi dans le groupe constitué par les extraits peptidiques de lupin ou « peptides de lupin », tels que ceux décrits dans la demande de brevet FR 2 792 202 déposée le 19 avril 1999 au nom de la société Laboratoires Pharmascience. On peut notamment citer l'extrait peptidique décrit dans la demande FR 2 792 202 sous la dénomination extrait B (LU105). Selon un autre mode avantageux de réalisation, ledit inhibiteur des MMPs est choisi dans le groupe constitué par les rétinoïdes.

Selon un mode de réalisation particulier de l'invention, la composition peut également comprendre en outre au moins un composé choisi dans le groupe constitué par les inhibiteurs de PKC, les agents anti-inflammatoires, les agents apaisants, les immunosuppresseurs, les chélateurs d'ions, les oxazolines, les alcanolamides et les dérivés d'acide carbamique. Ce ou ces composé(s) choisi(s) dans le groupe constitué par les inhibiteurs de PKC, les agents anti-inflammatoires, les agents apaisants, les immunosuppresseurs, les chélateurs de métaux, les oxazolines, les alcanolamides et les dérivés d'acide carbamique permet(tent) de jouer sur et/ou de limiter la réaction irritative ou de sensibilisation voire pour certains d'entre eux également d'inhiber la migration des cellules dendritiques, plus particulièrement des cellules de Langerhans, des dendrocytes dermiques, des monocytes, des lymphocytes, des kératinocytes, des mastocytes et des cellules endothéliales.

Par « PKC » ou « Protéines Kinases C », on entend au sens de la présente invention les enzymes qui catalysent une réaction de phosphorylation sur un substrat cellulaire.

Lorsqu'elles sont activées, les PKC phosphorylent des résidus sérine ou thréonine spécifiques sur des substrats protéiques, qui varient selon le type cellulaire. Dans de nombreuses cellules, l'activation des PKC augmente la transcription de gènes spécifiques.

Les protéines kinases C (PKC) sont des protéines codées par une famille de gènes (11 isoformes différentes). On sait notamment que ces protéines sont impliquées dans la transduction de signaux extracellulaires médiés par les facteurs de croissance, les cytokines, ainsi que par un certain nombre d'autres molécules biologiques. La protéine kinase β2 (PKC-β2) apparaît exprimée spécifiquement par les CL de l'épiderme.

Tout composé connu de l'homme du métier comme inhibant l'activité de phosphorylation des PKC peut ainsi être utilisé en tant que composé inhibiteur des PKC selon la présente invention. On peut par exemple citer les polypeptides décrits dans la demande WO 99/43805 (Incyte Genomics Inc.).

En particulier, le composé inhibiteur des PKC est choisi dans le groupe constitué par les inhibiteurs non spécifiques des PKC, les inhibiteurs spécifiques de l'isoforme PKC-β2, et les associations de ceux-ci.

Plus particulièrement, le composé inhibiteur des PKC est choisi dans le groupe constitué par les composés phénoliques et polyphénoliques, les procyanidines (catéchines, épicatéchines, etc.), l'alpha-amyrine, le lupéol, le lupéol linoléate, les stérols, les stanols, les alcools triterpéniques et leurs homologues hydrogénés, les antibiotiques tels que la staurosporine, Ro-318425 (ou 2-(8)-(aminométhyl)-6,7,8,9-tétrahydropyridol(1,2-a)indol-3-yl)3-(1-méthyl-indol-3-ylmaléimide, HCl) tel que commercialisé par la société Calbiochem, les composés qui agissent par compétition avec les activateurs physiologiques des PKC tels que le diacylglycérol et le phorbol ester, les lipides cutanés de type (lyso)sphingolipides, lysophospholipides tels que les céramides et pseudocéramides, sphingosines et phytosphingosines, les sphinganines, les dérivés, précurseurs, analogues et homologues de ces composés, d'origine naturelle ou synthétique.

Par « composés phénoliques et polyphénoliques », on entend selon l'invention les phénols simples, les benzoquinones, les acides phénoliques, les acétophénones, les acides phénylacétiques, les acides hydroxycinnamiques, les coumarines et isocoumarines, les chromones, les naftoquinones, les xanthones, les anthraquinones, les flavonoides, les lignanes et néolignanes, les lignines, les chalcones, les dihydrochalcones, les aurones, les flavones, les flavonols, les dihydroflavonols, les flavanones, les flavanols, les flavandiols ou leucoanthocyanidines, les anthocyanidines, les isoflavonoides, les biflavonoides, les proanthocyanidines et les tanins condensés.

Par "stérols", on entend plus particulièrement selon l'invention le stérol, c'est à dire le composé perhydro-1,2-cyclopentanophenanthrène ayant un groupe hydroxyle à la position 3, et les analogues du stérol de formule générale (I) ci-dessous.

Ainsi, de préférence, les stérols utilisables selon l'invention répondent à la formule générale suivante : 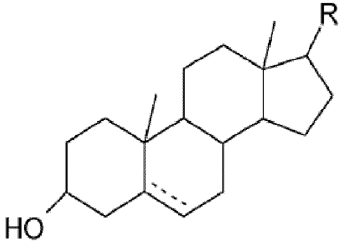 dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en C₁-C₁₂ les groupes alcoxy en C₁-C₈, les groupes alcényle en C₂-C₈, les groupe alcynyles en C₂-C₈, les groupes cycloalkyle en C₃-C₈, les groupes alcényle en C₂-C₈ halogénés, les groupes alcynyles en C₂-C₈ halogénés. Le terme "halogéné" désigne un ou plusieurs substituant halogène, à savoir un ou plusieurs atome(s) de chlore, fluor, brome ou iode.

Parmi les stérols pouvant être avantageusement utilisés selon l'invention, on peut citer en particulier le β-sitostérol, l'α-sitostérol, le γ-sitostérol, le stigmastérol, le campestérol ou encore le brassicastérol et les mélanges de ceux-ci. Par exemple, le β-sitostérol peut être utilisé sous la forme du produit dénommé "Ultra" (comprenant principalement du β-sitostérol) tel que commercialisé par la société Kaukas. Dans le cas d'une utilisation d'un mélange de stérols, on peut citer par exemple le produit dénommé "Generol" comprenant principalement du β-sitostérol (environ 50 % en poids), du stigmastérol, du brassicastérol et du campestérol tel que commercialisé par la société Cognis ou encore le produit "Primal" de la société Kaukas.

Parmi les alcools triterpéniques pouvant être avantageusement utilisés selon l'invention, on peut citer en particulier la β-amyrine, l'érythrodiol, le taraxastérol, le cycloarténol, le 24-méthylènecycloartanol, le lupéol, le lanostérol et les mélanges de ceux-ci.

Par "homologues hydrogénés" d'un alcool triterpénique, on entend selon l'invention le ou les composés alcool(s) triterpénique(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Plus particulièrement encore, le composé inhibiteur des PKC est choisi dans le groupe constitué par les sphingolipides et les lysophospholipides, comme ceux cités dans le tableau suivant :

**Tableau 1 : structures de sphingolipides et lysosphingolipides**

| | SPHINGOLIPIDES | LYSOSPHINGOLIPIDES |
|---|---|---|
| Structure | 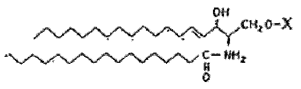 | 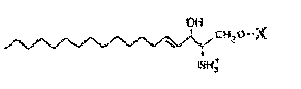 |
| X : H - | Ceramide | Sphingosine |
| X : Galactose - | Galactocerebroside | Psychosine (Galactosylsphingosine) |
| X : Sultogalactose - | Sulfolide | Lysosulfotide (Sulfogalactosylsphingosine) |
| 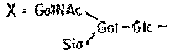 | GM₂ | Lyso GM₂ |
| X : Phosphorylcholine - | Sphingomyelin | Lysosphingomyelin |

On peut citer également plus particulièrement, comme composé inhibiteur des PKC, les lipides cutanés de type sphingolipides et lysophospholipides.

Comme sphingolipides, on peut citer ceux parmi les plus élémentaires tels que la sphingosine (D érythro dihydroxy 1,3 amino 2 octadécène 4t) et ses isomères, la phytosphingosine (D ribo trihydroxy 1,3,4 amino 2 octadécane) et ses isomères. Mais aussi, les lysosphingolipides (parmi eux, la lysosulfatide et la psychosine), la sulfogalactosylsphingosine, la sphinganine (2-amino 1,3 octadécane diol) et les sphingomyelines.

Comme phospholipides, on peut citer ceux parmi les familles des phosphatidylamino-alcools et des phosphatidylpolyols. Le groupe des phosphatidylamino-alcools comprend notamment les phosphatidylethanolamines (ou phosphatidylco lamines), les phosphatidylcholines, les phosphatidylsérines, les N-acylphosphatidylethanolamines. Celui des phosphatidylpolyols comprend quant à lui, les phosphatidylcholinositols, les diphosphoinositides, les lysodiphospho-inositides, les phosphatidylglycérols et les cardiolipides.

On peut citer également plus particulièrement, comme composé inhibiteur des PKC, les céramides, notamment les céramides du ciment intercornéocytaire de l'épiderme ainsi que les précurseurs des céramides que sont la sphingosine et la phytosphingosine.

D'une manière générale, les céramides peuvent être synthétisés chimiquement (on parle notamment de pseudo-céramides), être d'origine animale (des concentrations relativement élevées de sphingolipides sont présentes dans l'encéphale et la colonne vertébrale des mammifères), d'origine végétale (principalement des cérébrosides et autres sphingolipides glycosylées) ou encore être des dérivés de levures (configuration stéréochimique identique à celle des céramides naturellement présents dans la peau humaine).

Les céramides du ciment intercornéocytaire de l'épiderme peuvent être séparés par les méthodes classiques (chromatographie sur couche mince) en six fractions, correspondant à des composés différant par la nature des acides gras et la nature de la base impliquée (sphingosines, insaturées, ou phytosphingosines, saturées). Le tableau 2 suivant illustre les structures respectives présentes dans ces fractions, selon la classification de Werts et Downing. La fraction 6 peut elle-même être subdivisée par des méthodes plus fines en deux entités : les céramides 6a et 6b.

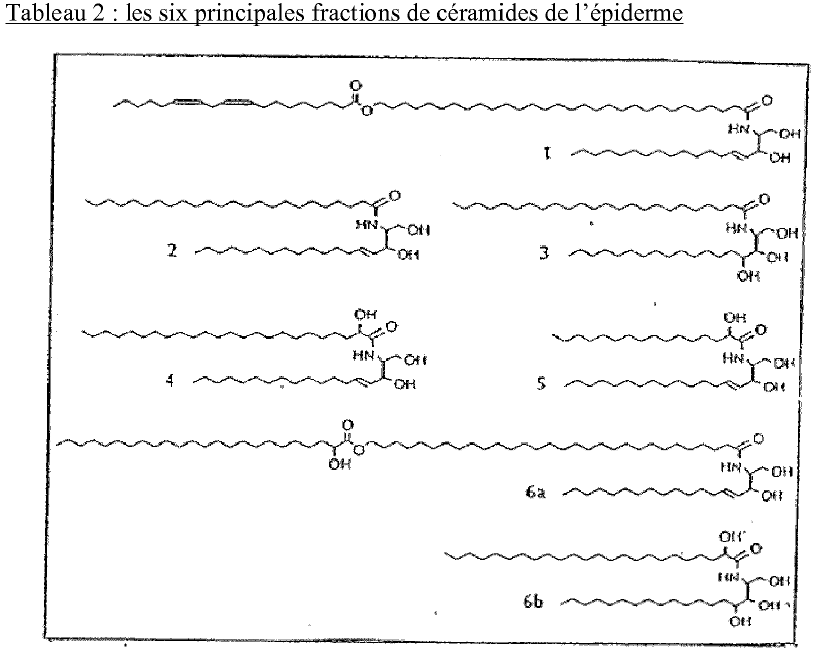

Ainsi, les céramides 1, les moins polaires, comportent une structure tout à fait particulière, que l'on retrouve dans le céramide 6a : un oméga-hydroxyacide à longue chaîne amidifiant la base, et attaché à son extrémité omega par une liaison ester à un autre acide gras (0-acylcéramides). Dans le cas de la fraction 1, les acides gras liés à la sphingosine sont essentiellement en C24, C26, C30, C32 ou C34, pouvant être saturés, monoéthyléniques (principalement pour les C30, C32 et C34) ou diéthyléniques (C32 et surtout C34). Quant à l'acide gras attaché à l'extrémité oméga du précédent, il s'agit, de façon largement prédominante pour les céramides 1, de l'acide linoléique, le rôle capital dans la fonction de barrière hybride de l'épiderme est bien connu.

La fraction 2, de structure plus classique (sphingosines ou dihydrosphingosines liées par une liaison amide à un acide gras, principalement C20 à C28), est la plus abondante.

La fraction 3 est assez similaire, la différence portant sur la nature de la base, qui, en l'occurrence, est essentiellement représentée par les phytosphingosines, saturées.

Les fractions 4 et 5 sont essentiellement caractérisées par la présence d'alpha-hydroxyacides liés à une sphingosine.

La fraction 6b est proche des fractions 4 et 5, comportant un alpha hydroxyacide, mais lié à une phytosphingosine, saturée.

La fraction 6a, comme le céramide 1, comporte le motif caractéristique que l'on ne retrouve qu'au niveau des céramides de l'épiderme, c'est-à-dire la liaison ester entre l'hydroxyle en oméga d'un acide gras lié à une sphingosine, et le groupement carboxylique d'un acide gras terminal, qui, cette fois, n'est pas l'acide linoléique, mais un alpha-hydroxyacide.

Il convient également de citer les phytocéramides (céramides à base phytosphingosine), les cholestérol-céramides synthétiques, les galacto ou gluco cérébrosides.

Enfin, parmi les composés inhibiteurs des PKC pouvant être utilisés selon la présente invention, la sphingosine est présente à l'état naturel dans la peau, et joue entre autre un rôle important dans fonction barrière du *stratum corneum,* en tant que précurseur des sphingolipides (céramides et sphingoglycolipides). Elle peut être dérivée de source biologique telle que des extraits de cerveaux bovins ou par voie de synthèse, à partir de la serine, comme décrit par exemple dans l'article de Newman, J.Am. CHEM., 95 (12) : 4098 (1973). On peut plus particulièrement citer les formes isomères de la sphingosine : D-érythro, L-thréo, L-érythro et D-thréo. La forme D-erythro est la plus fréquemment présente dans la nature.

Selon la présente invention, les composés inhibiteurs des PKC pouvant être utilisés comprennent les isomères, les dérivés (sels, complexes, etc.), les analogues, les homologues, les précurseurs et les métabolites des composés inhibiteurs des PKC décrits ci-dessus.

Les agents anti-inflammatoires pouvant être utilisés dans le cadre de la présente invention en association avec les oxazolidinones et les inhibiteurs de MMP sont avantageusement des agents anti-inflammatoires stéroïdiens (AIS), tels que les corticoïdes, ou non-stéroïdiens (AINS).

Les agents apaisants pouvant être utilisés dans le cadre de la présente invention en association avec les oxazolidinones et les inhibiteurs de MMP sont avantageusement des dérivés de réglisse et des dérivés de l'alpha-bisabolol.

Les immunosuppresseurs pouvant être utilisés dans le cadre de la présente invention en association avec les oxazolidinones et les inhibiteurs de MMP sont avantageusement le tacrolimus, le pimécrolimus, et la cyclosporine.

Les chélateurs d'ions pouvant être utilisés dans le cadre de la présente invention en association avec les oxazolidinones et les inhibiteurs de MMP sont avantageusement des chélateurs chimiques avantageusement choisis dans le groupe constitué par l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'acide diéthylènetriamine pentacétique (DTPA), et leurs mélanges. Les chélateurs d'ions peuvent également être des chélateurs biologiques avantageusement choisis dans le groupe constitué par la métallothionéine, la transférine, la lactoférine, la calmoduline, le chitosane méthylène phosphonate, et leurs mélanges.

Les ions chélatés sont avantageusement les ions Na⁺, K⁺, Ca²⁺, Cl⁻, Ni²⁺, Co⁺, Co²⁺, Zr ²⁺, Zr⁴⁺, mais aussi les ions chrome au niveau d'oxydation II et III tels que Cr²⁺, Cr³⁺, et Cr₂O₇²⁻.

Les oxazolines pouvant être utilisées dans le cadre de la présente invention en association avec les oxazolidinones et les inhibiteurs de MMP sont avantageusement des oxazolines répondant aux formules générales suivantes: 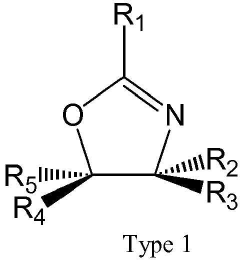 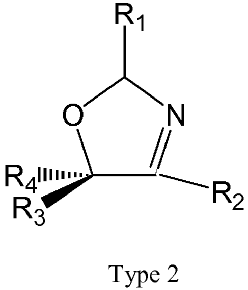 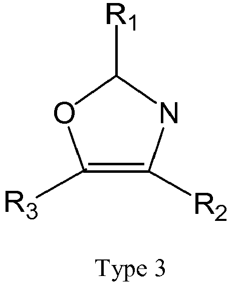 dans lesquelles R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) ; R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone.

Avantageusement selon la présente invention, ladite oxazoline est une oxazoline de type 1 choisie dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100, de formule :

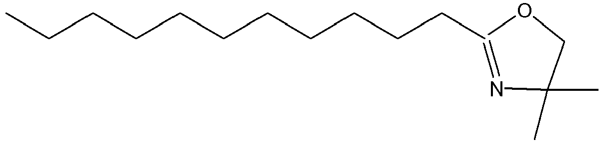

Les alcanolamides pouvant être utilisés dans le cadre de la présente invention en association avec les oxazolidinones et les inhibiteurs de MMP sont avantageusement des alcanolamides répondant à la formule générale suivante: 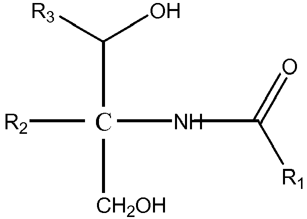 dans laquelle R₁ représente un groupe alkyle en C₁-C₄₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆); R₂ et R₃ représentent, de manière indépendante, un atome d'hydrogène, un groupe méthyle, un groupe alkyle en C₂-C₂₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone. Avantageusement selon l'invention, le radical R₁ représente un groupe alkyle comprenant de 1 à 40 atomes de carbone (en C₁-C₄₀), de manière préférée 6 à 22 atomes de carbone (en C₆-C₂₂) et de manière encore plus préférée de 8 à 18 atomes de carbone (en C₈-C₁₈), éventuellement saturé puisqu'il comprend de 0 à 6 insaturations, et comprenant de manière éventuelle au moins un radical hydroxyle, alkoxy ou alkoxycarbonyle tels que définis ci-dessus. De manière encore plus avantageuse selon la présente invention, ledit alcanolamide est l'alcanolamide appelé AK100 de formule :

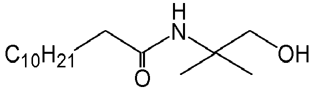

Les oxazolines et les alcanolamides selon la présente invention sont des agents inhibiteurs connus de la migration des cellules de Langerhans (Cf. demandes de brevet FR 2 834 213 et FR 2 834 216).

Les dérivés d'acide carbamique pouvant être utilisés dans le cadre de la présente invention en association avec les oxazolidinones et les inhibiteurs de MMP sont avantageusement des dérivés d'acide carbamique de formule générale suivante : 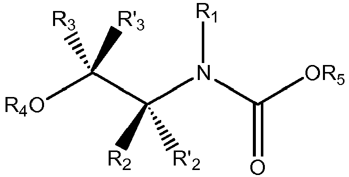 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation de l'invention, R₁ représente un atome d'hydrogène.

Selon un autre mode de réalisation de l'invention, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et/ou R'₂ représente un atome d'hydrogène.

Selon un autre mode de réalisation de l'invention, R₃ et R'₃ représentent un atome d'hydrogène.

Selon un autre mode de réalisation de l'invention, R₄ et R₅ représentent un atome d'hydrogène.

Dans un mode de réalisation particulier de la présente invention, R₁ représente un atome d'hydrogène, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et R'₂, R₃, R'₃, R₄ et R₅ représentent un atome d'hydrogène.

Avantageusement selon la présente invention, le dérivé d'acide carbamique est choisi dans le groupe constitué par le (1-Hydroxyméthyl-tridécyl)-acide carbamique et le (1-Hydroxyméthyl-undécyl)-acide carbamique.

Le (1-Hydroxyméthyl-tridécyl)-acide carbamique peut être représenté par la formule suivante :

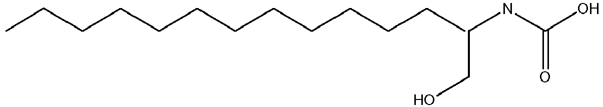

Les dérivés d'acide carbamique selon la présente invention sont des agents connus de la migration des cellules de Langerhans (cf. demande de brevet FR0214792).

Avantageusement selon la présente invention, la concentration en oxazolidinone est comprise entre environ 0,001 et environ 10 % en poids, et plus particulièrement entre environ 0,01 et 3 % en poids, avantageusement entre 0,1 et 1 % en poids, de manière encore plus avantageuse entre 0,1 et 0,5 % en poids, par rapport au poids total de la composition appliquée.

La composition peut être formulée sous la forme de différentes préparations adaptées à une administration topique, à une administration orale ou rectale, à une administration parentérale. De préférence, les différentes préparations sont adaptées à une administration topique et incluent les crèmes, les pommades, les lotions, les huiles, les patchs, les sprays ou tout autre produit pour application externe. Les modes d'administration, les posologies et les formes galéniques optimales des composés et compositions peuvent être déterminés selon les critères généralement pris en compte dans l'établissement d'un traitement cosmétique de préférence dermatologique, adapté à un patient comme par exemple l'âge ou le poids corporel du patient, la gravité de son état général, la tolérance au traitement, les effets secondaires constatés, et le type de peau. En fonction du type d'administration souhaité, la composition et/ou les composés actifs selon l'invention peuvent en outre comprendre au moins un excipient cosmétiquement acceptable, notamment dermatologiquement acceptable. De préférence, on utilise un excipient adapté pour une administration par voie topique externe. La composition peut en outre comprendre au moins un adjuvant cosmétiquement ou pharmaceutiquement connu de l'homme du métier, choisi parmi les épaississants, les conservateurs, les parfums, les colorants, les filtres chimiques ou minéraux, les agents hydratants, et les eaux thermales.

La composition et les composés actifs selon l'invention sont destinés à une utilisation en cosmétologie. La présente invention concerne une méthode de traitement cosmétique des peaux et/ou des muqueuses sensibles, intolérantes, , âgées, présentant des rougeurs cutanées, ou présentant un déséquilibre immunologique non pathologique lié au vieillissement intrinsèque, extrinsèque ou hormonal, caractérisée en ce qu'on applique sur la peau et/ou les muqueuses au moins une oxazolidinone de formule générale (I) telle que définie précédemment. Le composé actif choisi dans le groupe des oxazolidinones telles que définies précédemment peut également être appliqué en association avec au moins un autre composé sélectionné dans le groupe constitué par les inhibiteurs de métalloprotéases matricielles (MMPs), les inhibiteurs de PKC, les agents anti-inflammatoires, les agents apaisants, les immunosuppresseurs, et les chélateurs d'ions tels que définis précédemment. Dans le cadre de la méthode de traitement cosmétique selon la présente invention, le déséquilibre immunologique non pathologique est un déséquilibre temporaire ou non de la fonction immunitaire de la peau sans caractère de gravité.

D'autres caractéristiques et avantages de l'invention apparaissent dans la suite de la description avec les exemples représentés ci-après. Les exemples suivants sont destinés à illustrer l'invention sans aucunement en limiter la portée. A moins qu'il n'en soit précisé autrement, les pourcentages indiqués dans les exemples suivants sont des pourcentages en poids. La 4-dodécyl-oxazolidin-2-one est dénommée ci-après oxazolidinone A, la 4,5-didodécyl-oxazolidin-2-one est dénommée ci-après oxazolidinone B, et la 3,4-didodécyl-oxazolidin-2-one est dénommée ci-après oxazolidinone C.

### Exemple 1 : Exemples de compositions selon la présente invention

| | % en poids |
|---|---|
| Eau | 58.1 |
| Polydécène hydrogéné | 12 |
| Glycérine | 15 |
| Carbonate de dicaprylyle | 7 |
| Glucoside de lauryle | 2,5 |
| Polyglycéryl-2 dipolyhydroxystéarate | 3,5 |
| Extrait peptidique de lupin (Protéine hydrolysée) | 0,2 |
| Acrylate/Copolymère d'acrylate d'alkyle en C₁₀-₃₀ | 0,4 |
| Hydroxyméthylglycinate de sodium | 0,4 |
| Gomme de xanthane | 0,3 |
| Oxazolidinone A ou B ou C | 0,5 |
| Hydroxyde de sodium | 0,07 |
| Acide citrique | 0,03 |

### Exemple 2 : Mesure de la perte insensible en eau de compositions selon la présente invention:

Les mesures de la Perte Insensible en Eau (P.I.E.) permettent de vérifier l'intégrité de la fonction "barrière" du stratum corneum, en quantifiant les échanges d'eau entre le milieu environnant et la peau. Une détérioration de l'intégrité du stratum corneum se traduit par une augmentation de la P.I.E. liée à une plus grande perméabilité du tégument. Il a ainsi été démontré que des volontaires ayant une peau de nature sèche auraient une peau qui équilibrerait moins d'eau et à laquelle serait associée une élévation de la P.I.E. Par extension, un produit présentant un pouvoir filmogène, et donc un effet barrière, limiterait de façon significative la P.I.E.

### Mode opératoire :

La Perte Insensible en Eau a été déterminée après application cutanée unique des 3 formules présentées dans l'exemple ci-dessus, contenant l'oxazolidinone A, B ou C, chez un volontaire adulte de sexe féminin ayant une peau des jambes sèche.
- Mesures de P.I.E. : Les mesures de P.I.E. sont effectuées en mode standard. La sonde est positionnée sur la zone de mesure cutanée, sans pression. Un signal sonore se déclenche pour signifier que la mesure commence. Une mesure de P.I.E. est prise toutes les 2 secondes pendant 2 minutes environ, un temps de 1 minute étant en moyenne nécessaire pour obtenir des valeurs stables. La valeur moyenne des 20 dernières secondes et la déviation de la valeur moyenne s'affiche alors à l'écran.
- Zones : Quatre zones cutanées de 30 cm² (6 cm x 5 cm) environ de la face externe des jambes sont délimitées entre le genou et la cheville. Trois zones sont traitées respectivement par les trois formules présentées ci-dessus, contenant l'oxazolidinone A, B ou C, et une quatrième zone constitue la zone témoin sur laquelle aucun produit n'est appliqué. La zone témoin et les trois zones traitées ont été appliquées sur les jambes droite ou gauche, selon une randomisation au hasard.

Les mesures ont été réalisées, pour chaque temps de l'étude et pour chaque zone, sur une sous-zone de 10 cm² (une sous-zone par temps de mesure), après un essuyage délicat à l'aide d'un papier en ouate de cellulose.
- Dose appliquée : 0,06 g environ de chaque produit sur l'ensemble des quatre zones, soit 2 ± 0,04 mg/cm².
- Modalités d'application : un massage délicat, de manière uniforme, est effectué à l'aide d'un doigtier, afin d'assurer la meilleure pénétration possible du produit.
- Période de repos : 30 minutes minimum avant les mesures expérimentales initiales.
- Temps de mesure : T0 (avant l'application), T1 heure et T8 heures.
- Panel : 10 panélistes ont été admis par le Chargé d'Etude et ont participé à la totalité de l'essai. L'analyse des résultats a donc porté sur un panel de 10 volontaires, de sexe féminin, âgées de 22 à 63 ans (moyenne : 39 ans).

### Résultats :

Le Tableau 3 rassemble les résultats portant sur l'étude de l'évolution de la Perte Insensible en Eau (P.I.E.) des Formules 1 (oxazolidinone A à 0.5%); 2 (oxazolidinone B à 0.5%) et 3 (oxazolidinone C à 0.5%) en fonction du temps.

**Tableau 3 :**

| | P.I.E. (g / m².h) | | | |
|---|---|---|---|---|
| Zones | Placebo Zone 1 | Formule 1 Zone 2 | Formule 2 Zone 3 | Formule 3 Zone 4 |
| T0 | 3,5 | 3,4 | 3,5 | 3,4 |
| T 1 heure | 3,3 | 3,2 | 3,3 | 3,3 |
| T 8 heures | 3,2 | 3,3 | 3,3 | 3,2 |

En conclusion, la perte insensible en eau n'est quasiment pas modifiée après application des formules 1, 2 et 3 contenant respectivement 0.5 % des oxazolidinones A, B et C. Par conséquent, les formules 1, 2 et 3 à base d'oxazolidinones n'ont donc aucune propriété filmogène et aucun effet barrière. Elles ne peuvent donc pas contribuer à bloquer la pénétration éventuelle d'agents allergènes ou sensibilisants.

### Exemple 3 : Etude de l'activité des oxazolidinones A, B et C sur l'inhibition de la migration des CL fraîchement isolées à partir de fragments de peau humaine :

### 1. Matériel et méthodes

### 1.1 Obtention des cellules de Langerhans

Des suspensions de cellules épidermiques ont été obtenues par traitement enzymatique (0,05% trypsine, pendant 18h à +4°C) de fragments de peau humaine normale issus de chirurgie plastique. Les suspensions obtenues contiennent en moyenne 2 à 4% de CL. L'obtention de suspensions contenant en moyenne 70% de CL est basée sur le principe de la centrifugation sur gradient de densité (Lymphoprep™) et élimination des kératinocytes.

### 1.2 Préparation des milieux

Le milieu de base choisi pour l'ensemble de l'étude a été le RPMI 1640 (Gibco BRL, France). Les oxazolidinones A, B et C, fournies par EXPANSCIENCE, à la concentration de 10⁻² M en solution dans du DMSO (Diméthyl Sulfoxide), ont été diluées en RPMI-1640 et testées à 1 µM.

### 1.3 Sensibilisation des CL

On a utilisé comme agent sensibilisateur le DNSB (Sigma Aldrich), forme soluble du DNCB (dinitro-chloro-benzène), solubilisé en RPMI-BSA et utilisé à la concentration de 50 µM.

### 1.4 Migration des CL

Un système de chambre de culture à deux compartiments (Falcon, Becton Dickinson, France) a été utilisé. Le compartiment supérieur est séparé du compartiment inférieur par une membrane de porosité 8 µm, sur laquelle sont déposées 50 µg/cm² de Matrigel. La membrane est alors recouverte de protéines formant un film équivalent à une membrane basale (laminine, collagène IV, nidogène, entactine, héparane sulfate protéoglycanes). Les cellules reprises dans le milieu RPMI-BSA seul ou en présence des différents produits sont déposées dans le compartiment supérieur. Dans le compartiment inférieur, est ajouté du surnageant de culture de fibroblastes humains normaux. Après 18h d'incubation à 37°C, le nombre de cellules vivantes ayant traversé le Matrigel et se trouvant dans le compartiment inférieur est compté sous microscope (les CL sont facilement identifiables par leur forme dendritique). Chaque essai est réalisé en triplicate.

### 2. Résultats

Les résultats portant sur la migration des CL sont présentés dans le tableau 4 suivant.

**Tableau 4 :**

| | Cellules contrôle | Cellules sensibilisées par l'haptène DNSB | DNSB + oxazolidinone A (1 µM) | DNSB + oxazolidinone B (1 µM) | DNSB + oxazolidinone C (1 µM) |
|---|---|---|---|---|---|
| Index de migration | 1 | 2,13 | 1,2 | 0,98 | 1,1 |

Le tableau 4 montre ainsi le rapport entre le nombre de cellules ayant migré en présence de DNSB +/- le produit à l'essai et le nombre de cellules ayant migré dans les conditions normales (cellules contrôle non sensibilisées et non traitées). Les CL fraîchement isolées de l'épiderme n'ont pas une capacité migratoire élevée. Dans l'expression des résultats, la capacité migratoire des CL contrôle (non traitées et non sensibilisées) est arbitrairement fixée à 1.

Le traitement des cellules avec l'haptène DNSB a stimulé la migration des CL de façon significative (+113%) par rapport aux cellules normales non stimulées (cellules contrôle).

Les oxazolidinones A, B et C à la concentration de 1 µM inhibent de manière significative la migration des CL induite par le DNSB. Les cellules ainsi traitées ont un index de migration comparable à celui des cellules contrôle non sensibilisées.

En conclusion, il a ainsi été montré, de manière tout à fait surprenante, qu'en utilisant des CL fraîchement isolées, placées dans un système de chambre de culture à deux compartiments (permettant la migration cellulaire), que les oxazolidinones A, B et C inhibent de manière significative la migration des CL.

### Exemple 4 : Etude de l'activité de l'association de l'oxazolidinone B (4,5-didodécyl oxazolidin-2-one) et du LU105 (inhibiteur de MMPs) sur l'inhibition de la migration des CL fraîchement isolées à partir de fragments de peau humaine

Afin d'illustrer l'intérêt de l'association entre une oxazolidinone et un inhibiteur de MMPs, l'oxazolidinone B (4, 5-didodécyl oxazolidin-2-one), ainsi que le LU105 (LU 105 est un inhibiteur de MMPs, correspondant à un extrait peptidique de lupin blanc commercialisé par la Société Expanscience sous le nom de marque Actimp 193®) ont été appliqués simultanément sur des CL fraîchement isolées.

### 1. Matériel et méthodes

Idem que l'exemple 3.

### 2. Résultats

Les résultats portant sur la migration des CL sont présentés dans le tableau 5 suivant.

**Tableau 5 :**

| | Cellules contrôle | Cellules sensibilisées par l'haptène DNSB | DNSB + oxazolidinone B (1 µM) | DNSB + LU105 (5 µM) | DNSB + oxazolidinone B (1 µM) + LU105 (5 µg/ml) |
|---|---|---|---|---|---|
| Index de migration | 1 | 1,78 | 1,2 | 1,3 | 0,91 |

En conclusion, les deux molécules à l'essai (oxazolidinone B et LU105) testées séparément inhibent de manière significative la migration des CL sensibilisées. Lorsque ces deux molécules sont associées, leur activité respective est potentialisée, et les cellules sensibilisées ont une capacité migratoire proche de celle des cellules normales non sensibilisées. En d'autres termes, l'oxazolidinone B et LU105 agissent en synergie pour inhiber la migration des CL activées.

### Etude de l'activité de l'oxazolidinone B sur l'inhibition de la migration des cellules dendritiques sur la souris

### 1. Matériels & méthodes

### 1.1 Réactifs

Le FITC (Fluorescéine isothiocyanate, Sigma, St. Louis, MO) a été dilué extemporanément dans un mélange acétone : dibutylphthalate (1:1).

### 1.2 Inhibiteurs

L'oxazolidinone B et le LU105 (LU 105 est un inhibiteur de MMPs, correspondant à un extrait peptidique de lupin blanc commercialisé par la Société Expanscience sous le nom de marque Actimp 193®) ont été fournis par les "Laboratoires Expanscience", et formulés seuls ou en association dans un véhicule inerte compatible avec une application topique, tel que décrit à l'exemple 1 ci-dessus [oxazolidinone B (0,5%) ± LU105 (2%)]. Les différentes formulations ont été appliquées sur les oreilles de souris deux fois par jour pendant 4 jours consécutifs. Trois heures après la dernière application, 1,5% de FITC sont appliqués sur les deux oreilles (l'une traitée et l'autre non traitée (Contrôle)).

### 1.3 Migration des cellules de Langerhans (CL) et des cellules dendritiques CD sur la souris

L'effet des deux molécules a été évalué in vivo chez la souris. La peau des deux oreilles est badigeonnée avec 1,5% FITC (2X5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon; Becton Dickinson), puis lavés. Les cellules sont ensuite centrifugées 10 min à 600 x g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules FITC+, PE+ et Cya+ sont comptabilisées car elles représentent la population de cellules ayant migré de la peau vers les GL suite à l'application de l'haptène.

### 2. Résultats

L'application topique du véhicule seul n'entraîne aucune modification du nombre de CD FITC+ au niveau des GL. Le véhicule est donc sans effet sur les capacités migratoire des CD.

Les résultats portant sur la migration des CD sont présentés dans le tableau 6 suivant.

**Tableau 6 :**

| | OXAZOLIDINONE B (0,5%) | LU105 (2%) | OXAZOLIDINONE B (0,5%) + LU105 (2%) |
|---|---|---|---|
| Inhibition de la migration en % | 28 | 30 | 90 |

La migration des CD au niveau des GL, suite à l'application de l'haptène FITC, est inhibée dans des proportions comparables et sans différence significative par l'oxazolidinone B à la dose de 0,5% et le LU105 à la dose de 2%.

Lorsque les deux types de molécules sont associés, cette inhibition est presque totale. En conclusion, il a ainsi été montré de manière tout à fait surprenante, en utilisant un modèle de souris sensibilisées par l'haptène FITC, que l'oxazolidinone B inhibe de manière significative la migration des CD vers les GL. Par ailleurs, l'oxazolidinone B et le LU105 agissent en synergie pour inhiber la migration des CD chez la souris sensibilisée.

## Revendications

1. Méthode de traitement cosmétique des peaux et/ou des muqueuses sensibles, intolérantes, âgées, présentant des rougeurs cutanées, ou présentant un déséquilibre présentant des rougeurs cutanées, ou présentant un déséquilibre immunologique non pathologique lié au vieillissement intrinsèque, extrinsèque ou hormonal, **caractérisée en ce qu'**on applique sur la peau et/ou les muqueuses au moins une oxazolidinone permettant d'inhiber la migration des cellules de Langerhans de formule générale (I) 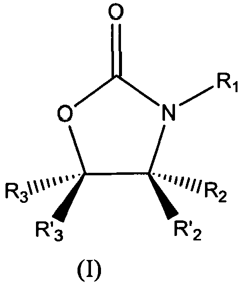 dans laquelle R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH); R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH); R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi qu'un ou plusieurs substituant(s) hydroxy (OH).

2. Méthode de traitement cosmétique selon la revendication 1, **caractérisée en ce qu'**on applique sur la peau et/ou les muqueuses au moins une oxazolidinone de formule générale (I) dans laquelle R₁ représente un atome d'hydrogène.

3. Méthode de traitement cosmétique selon les revendications 1 ou 2, **caractérisée en ce qu'**on applique sur la peau et/ou les muqueuses au moins une oxazolidinone de formule générale (I) dans laquelle R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et R'₂ représente un atome d'hydrogène.

4. Méthode de traitement cosmétique selon l'une quelconque des revendications 1 à 3, **caractérisée en ce qu'**on applique sur la peau et/ou les muqueuses au moins une oxazolidinone de formule générale (I) dans laquelle R₃ et R'₃ représentent un atome d'hydrogène.

5. Méthode de traitement cosmétique selon l'une quelconque des revendications 1 à 4, **caractérisée en ce que** ladite oxazolidinone est choisie dans le groupe constitué par la 4-dodécyl-oxazolidin-2-one, la 3,4-didodécyl-oxazolidin-2-one et la 4,5-didodécyl-oxazolidin-2-one.

## Patentansprüche

1. Kosmetische Behandlungsmethode der sensiblen, intoleranten, alten Haüte und/oder der Schleimhäute, die Hautrötungen oder ein immunologisches, nicht pathologisches, mit der intrinsischen, extrinsischen oder hormonalen Alterung verbundenes Ungleichgewicht aufweisen, **dadurch gekennzeichnet, dass** man auf die Haut und/oder die Schleimhäute mindestens ein Oxazolidinon aufträgt, das erlaubt, die Migration der Langerhans-Zellen zu hemmen, mit der allgemeinen Formel (I) 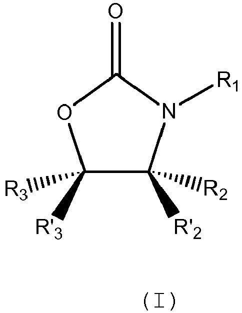 in der R₁ ein Wasserstoffatom oder eine lineare oder verzweigte, gesättigte oder ungesättigte C₁-C₃₀-Alkylgruppe darstellt, die eventuell eine oder mehrere ethylenisch und/oder acetylenisch ungesättigte Verbindungen umfasst sowie einen oder mehrere Hydroxy (OH) Substituenten; R₂ und R'₂ unabhängig ein Wasserstoffatom oder eine lineare oder verzweigte, gesättigte oder ungesättigte C₁-C₃₀-Alkylgruppe darstellen, die eventuell eine oder mehrere ethylenisch und/oder acetylenisch ungesättigte Verbindungen umfasst sowie einen oder mehrere Hydroxy (OH) Substituenten; R₃ und R'₃ unabhängig ein Wasserstoffatom oder eine lineare oder verzweigte, gesättigte oder ungesättigte C₁-C₃₀-Alkylgruppe darstellen, die eventuell eine oder mehrere ethylenisch und/oder acetylenisch ungesättigte Verbindungen umfasst sowie einen oder mehrere Hydroxy (OH) Substituenten.

2. Kosmetische Behandlungsmethode nach Anspruch 1, **dadurch gekennzeichnet, dass** man auf die Haut und/oder die Schleimhäute zumindest ein Oxazolidinon der allgemeinen Formel (I) aufträgt, in der R₁ ein Wasserstoffatom darstellt.

3. Kosmetische Behandlungsmethode nach den Ansprüchen 1 oder 2, **dadurch gekennzeichnet, dass** man auf die Haut und/oder die Schleimhäute zumindest ein Oxazolidinon der allgemeinen Formel (I) aufträgt, in der R₂ eine gesättigte lineare C₈-C₂₂-, in vorteilhafter Weise eine C₉-C₁₈-, in noch vorteilhafterer Weise eine C₉-C₁₂-, in noch vorteilhafterer Weise eine C₁₀-C₁₁-Alkylgruppe darstellt und R'₂ ein Wasserstoffatom darstellt.

4. Kosmetische Behandlungsmethode nach einem der Ansprüche 1 bis 3, **dadurch gekennzeichnet, dass** man auf die Haut und/oder die Schleimhäute zumindest ein Oxazolidinon der allgemeinen Formel (I) aufträgt, in der R₃ und R'₃ ein Wasserstoffatom darstellen.

5. Kosmetische Behandlungsmethode nach einem der Ansprüche 1 bis 4, **dadurch gekennzeichnet, dass** das Oxazolidinon aus der Gruppe ausgewählt ist, die von dem 4-Dodecyl-oxazolidin-2-on, dem 3,4-Didodecyl-oxazolidin-2-on und dem 4,5-Didodecyl-oxazolidin-2-on gebildet ist.

## Claims

1. Method for cosmetic treatment of skin and/or mucosa that is sensitive, intolerant or aged, or that exhibits skin redness or a non-pathological immunological imbalance related to intrinsic, extrinsic or hormonal aging, **characterized in that** one applies to the skin and/or mucosa at least one oxazolidinone for inhibiting the migration of Langerhans cells of general formula (I) 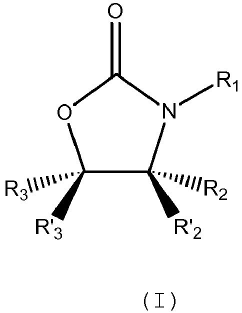 wherein R₁ represents a hydrogen atom or a linear or branched, saturated or unsaturated C₁-C₃₀ alkyl group, optionally comprising one or more ethylenic and/or acetylenic unsaturation(s), as well as one or more hydroxy (OH) substituent (s); R₂ and R'₂ independently represent a hydrogen atom or a linear or branched, saturated or unsaturated C₁-C₃₀ alkyl group, optionally comprising one or more ethylenic and/or acetylenic unsaturation(s), as well as one or more hydroxy (OH) substituent(s); R₃ and R'₃ independently represent a hydrogen atom or a linear or branched, saturated or unsaturated C₁-C₃₀ alkyl group, optionally comprising one or more ethylenic and/or acetylenic unsaturation(s), as well as one or more hydroxy (OH) substituent(s).

2. Method for cosmetic treatment according to claim 1, **characterized in that** one applies to the skin and/or mucosa at least one oxazolidinone of general formula (I) wherein R₁ represents a hydrogen atom.

3. Method for cosmetic treatment according to claims 1 or 2, **characterized in that** one applies to the skin and/or mucosa at least one oxazolidinone of general formula (I) wherein R₂ represents a linear saturated C₈-C₂₂, advantageously C₉-C₁₈, more advantageously C₉-C₁₂, even more advantageously C₁₀-C₁₁, alkyl group and R'₂ represents a hydrogen atom.

4. Method for cosmetic treatment according to any one of claims 1 to 3, **characterized in that** one applies to the skin and/or mucosa at least one oxazolidinone of general formula (I) wherein R₃ and R'₃ represent a hydrogen atom.

5. Method for cosmetic treatment according to any one of claims 1 to 4, **characterized in that** said oxazolidinone is selected from the group consisting of 4-dodecyl-oxazolidin-2-one, 3,4-didodecyl-oxazolidin-2-one and 4,5-didodecyl-oxazolidin-2-one.
